# EUROPÄISCHE PATENTANMELDUNG

(11) **EP 2 168 606 A1**
(43) Veröffentlichungstag der Anmeldung: **31.03.2010**
(21) Anmeldenummer: 09015307.3
(22) Anmeldetag: 16.01.2007
(51) Int. Cl.: A61L 2/18, A61B 19/00, A61C 19/00, A61L 2/025, A61L 2/24, A61L 101/22, A61L 101/54

(54) **Verfahren und Vorrichtung zur Aufbereitung eines medizinischen Instruments**

(62) Teilanmeldung aus: 07000808.1
(71) Anmelder: W & H Dentalwerk Bürmoos GmbH, 5111 Bürmoos (AT)
(72) Erfinder: Schaffarzick, 5061 Elsbethen (AT)
(74) Vertreter: Appelt, Christian W.

(57) **Zusammenfassung**

Die vorliegende Erfindung betrifft ein Verfahren zur Aufbereitung eines medizinischen Instruments, insbesondere eines Handstücks, das die folgenden Schritte umfaßt: Vorspülen des Instruments mit einer Vorspülflüssigkeit, Spülen des Instruments mit einem Flüssigkeitsgemisch, das Wasser und ein Reinigungsmittel umfaßt, Desinfizieren des Instruments mit einem Flüssigkeitsgemisch, das Wasser umfaßt, wobei die einzelnen Verfahrensschritte kontinuierlich durchgeführt werden und wobei das Trägermedium kontinuierlich zugeführt und abgeführt wird. Die Erfindung betrifft ferner eine Vorrichtung zur Durchführung eines solchen Verfahrens.

## Beschreibung

Die Erfindung bezieht sich auf ein Verfahren und eine Vorrichtung zur Aufbereitung von medizinischen, insbesondere dentalmedizinischen und chirurgischen Instrumenten, insbesondere Handstücken.

Instrumente und/oder Handstücke sollen möglichst nach jeder Verwendung gereinigt werden, sowohl aus hygienischen Gründen, um die Übertragung von Keimen, Krankheitserregern, Blut etc. zwischen Patienten zu verhindern, als auch aus technischen Gründen, da Partikel, zum Beispiel Gewebeteilchen, insbesondere Knochenmaterial, und Flüssigkeiten, zum Beispiel Behandlungsflüssigkeiten zur Kühlung der Präparationsstelle oder Körperflüssigkeiten des Patienten, während der Behandlung in die Instrumente oder Handstücke eindringen und dort Bauteile des Instruments oder des Handstücks, zum Beispiel Lager etc., beschädigen oder ihre Funktion beeinträchtigen können. Eine Reinigung der Instrumente dient auch der Entfernung alten Schmiermaterials, an das sich insbesondere Verunreinigungen festsetzen können.

Dokument WO 01/56615 offenbart ein Reinigungs- und Sterilisationssystem sowie ein entsprechendes Verfahren, bei dem Wasch- und Dekontaminationslösungen in einem Kreislauf auf ein zu reinigendes Gerät gesprüht werden, wobei dann die Lösungen wieder gesammelt und in den Kreislauf eingespeist und wieder auf das zu reinigende Gerät gesprüht werden.

Dokument DE 198 58 420 A1 offenbart ein Verfahren zum Reinigen und Sterilisieren eines medizinischen Geräts, wobei das Sterilisieren und Trocknen gleichzeitig durchgeführt wird. Beim Sterilisieren wird eine vorbestimmte Menge eines chemischen, keimtötenden Stoffs in dem Reinigungsbehälter verdampft, so dass das Gerät unter Vakuum sterilisiert, desinfiziert und getrocknet wird.

Dokument EP 1 186 574 A1 beschreibt ein Verfahren zur Elimination von Biofilmen in oberflächenflüssigkeitsführenden Systeme, wie z.B. in einem Wasserversorgungsspeicher oder in Wasserleitungssystemen oder dergleichen, wobei ein enzymatischer Wirkstoff verwendet wird.

Es ist daher eine Aufgabe der vorliegenden Erfindung, ein verbessertes Verfahren und eine entsprechende Vorrichtung zur Aufbereitung von medizinischen, insbesondere dentalmedizinischen und chirurgischen Instrumenten, insbesondere Handstücken, zur Verfügung zu stellen.

Diese Aufgabe wird durch ein Verfahren gemäß Anspruch 1 sowie eine Vorrichtung gemäß Anspruch 12 gelöst. Die Ansprüche 2 bis 11 betreffen besonders vorteilhafte Ausgestaltungen des Verfahrens gemäß Anspruch 1, die Ansprüche 13 bis 15 betreffen besonders vorteilhafte Ausführungsformen der Vorrichtung gemäß Anspruch 12.

Die vorliegende Erfindung bezieht sich allgemein auf ein Verfahren und eine Vorrichtung zur Aufbereitung eines medizinischen Instruments, wobei sie sich hauptsächlich auf Handstücke oder Winkelstücke bezieht, jedoch auch beliebige andere medizinische Instrumente aufbereitet werden können. Die nachfolgende Beschreibung bezieht sich daher immer sowohl auf Handstücke oder Winkelstücke im engeren Sinne, als auch auf medizinische Instrumente im weiteren Sinne, ohne jegliche einschränkende Wirkung, gleichwohl zur Vereinfachung teilweise explizit lediglich auf ein Handstück Bezug genommen wird.

Gemäß der Erfindung werden die einzelnen Verfahrensschritte in einem kontinuierlichen Durchlaufverfahren durchgeführt, wobei Wasser als Trägermittel kontinuierlich zugeführt, zum Vorspülen, Spülen und Desinfizieren des zu reinigenden Handstücks verwendet und auch kontinuierlich abgeführt wird, wobei dem Trägermittel Wasser während des Spülens das Reinigungsmittel, gemäß dem Anspruch 2 bevorzugt das enzymatische Reinigungsmittel, und während des Desinfizierens das Desinfektionsmittel, gemäß Anspruch 2 bevorzugt das Oxidationsmittel, zugeführt wird, bevor das Trägermedium auf das zu reinigende Handstück wirkt.

Dieses kontinuierliche Durchlaufprinzip hat insbesondere den Vorteil, daß das oder die zu reinigenden Handstücke nicht in einem Wasserbad verweilen müssen, dadurch keine erneute Kontamination auftreten kann, was insbesondere auch auf der Vorrichtungsseite den Vorteil hat, daß keine flüssigkeitsdichten oder druckdichten Behälter in der Vorrichtung erforderlich sind. Ferner sind außer den Lagerbehältern für die Reinigungsflüssigkeit, das Desinfektionsmittel und gegebenenfalls das Schmiermittel keine weiteren Behälter erforderlich. Auch benötigt ein solches System keine Sammelkammer zum Sammeln der Abwässer, da auch diese kontinuierlich abgeführt werden.

Besonders bevorzugt wird auch sichergestellt, daß keinerlei Kreislaufführung der Medien stattfindet, daher die Medien nur einmal verwendet werden, was ebenfalls sicherstellt, daß keinerlei Verunreinigungen, die bereits entfernt wurden, erneut an die Handstücke gelangen. Dies ermöglicht ein Reinigungsverfahren, das extrem hohen Anforderungen genügt.

Bei sämtlichen Schritten des erfindungsgemäßen Verfahrens werden die Flüssigkeiten im Durchlaufverfahren durch das Handstück oder durch mehrere Handstücke (oder ein Instrument) geführt, die gegebenenfalls gleichzeitig in einer entsprechenden Reinigungsvorrichtung eingesetzt sein können.

Die Kombination der Verfahrensschritte A, B und C führen in dieser Reihenfolge und unter den definierten Bedingungen zu außergewöhnlich guten Ergebnissen, wobei die Erfinder festgestellt haben, daß die hervorragenden Ergebnisse nicht nur auf die kumulierten Ergebnisse der Einzelschritte zurückzuführen sind, sondern sich eine besondere kombinatorische Wirkung ergibt, insbesondere im Hinblick auf die Verfahrensschritte B und C, nämlich das Spülen des Instruments mit einem, insbesondere enzymatischen, Reinigungsmittel in Verbindung mit dem nachfolgenden Desinfizieren des Handstücks, insbesondere unter Verwendung eines Oxidationsmittels:

Der Schritt A, das Vorspülen des Instruments, stellt sicher, daß das Instrument von groben Schmutzpartikeln und Verunreinigungen gesäubert wird, wobei bevorzugt eine maximale Temperatur gewählt wird, die sicherstellt, daß eine Koagulation von Eiweißen, die eine Reinigung erschweren würde, verhindert wird. Bevorzugt wird eine maximale Temperatur von 42°C gewählt, bei einem besonderen Verfahren wird eine maximale Temperatur von 40°C eingestellt.

Der nachfolgende, darauf aufbauende Schritt des Spülens des Instruments oder Handstücks mit einem Flüssigkeitsgemisch, das Wasser und ein bevorzugt enzymatisches, Reinigungsmittel umfaßt, stellt zum einen einen sehr effektiven Reinigungsschritt dar, mittels dem in kurzer Zeit sehr gute Ergebnisse erzielt werden, insbesondere viele Verunreinigungen von den Oberflächen des Handstücks entfernt werden können. Unter Reinigung im Sinne dieser Erfindung ist das Entfernen, die sogenannte "Abreicherung", von vorhandene Verunreinigungen, insbesondere von organischem Material inklusive Micro-Organismen, zu verstehen, wobei bei Schritt B gemäß dieser Erfindung sowohl eine mechanische Wirkung durch das Flüssigkeitsgemisch selbst erzielt wird als auch eine chemische oder auch biochemische Wirkung aufgrund des beigefügten, bevorzugt enzymatischen, Reinigungsmittels, bevorzugt gekoppelt mit einer thermischen Wirkung durch das Aufheizen des Flüssigkeitsgemischs auf 40°C bis 70°C. Die Reinigungsleistung, die mit einem Reinigungsschritt erzielt wird, kann mit Prüfmodellen überprüft werden, die beispielsweise in der Norm EN ISO 15883-1 beschrieben sind.

Zum anderen haben die Erfinder festgestellt, daß gerade das Spülen des Instruments oder Handstücks mit einem Flüssigkeitsgemisch, das ein, bevorzugt enzymatisches, Reinigungsmittel umfaßt, eine "Konditionierung" für den nachfolgenden Schritt des Desinfizierens mittels eines Oxidationsmittels hervorruft, so daß ein Gesamtergebnis ermöglicht wird, das über die zu erwartende "Summe" der Ergebnisse der Einzelschritte hinausgeht. Die durch diese "Konditionierung" durch das, bevorzugt enzymatische, Reinigungsmittel hervorgerufene kombinatorische Wirkung im Zusammenhang mit dem zur Desinfektion bevorzugt verwendeten Oxidationsmittel führt daher zu noch deutlich besseren Ergebnissen.

Die Erfinder haben festgestellt, daß bei einer alternativen Reinigung in Schritt B ohne ein enzymatisches Reinigungsmittel und bei ansonsten unveränderten Verfahrensschritten das Desinfizieren der Handstücke zu schlechteren Ergebnissen, also zu einer höheren Rest-Keimzahl, führt, selbst wenn das Reinigungsergebnis bzw. die Reinigungsleistung nach Schritt B auf gleichem Niveau gehalten wird, was beispielsweise durch die oben genannten Prüfmodelle gemäß EN ISO 15883-1 geprüft werden kann. Ebenso führt es zu einem schlechteren Gesamtergebnis, wenn der Schritt C gemäß der Erfindung durch einen anderen Desinfektionsschritt ersetzt würde, beispielsweise durch eine Desinfektion mittels thermischer Behandlung.

Erfindungsgemäß werden die Flüssigkeiten im Durchlaufverfahren auf oder durch das Instrument, insbesondere durch das Handstück geführt, was den Vorteil hat, daß die Instrumente nicht längere Zeit in einem Flüssigkeitsbad verbleiben brauchen, so daß vermieden wird, daß sich beispielsweise bereits losgelöste Verschmutzungen wieder an einer anderen Stelle des Instruments ablagern. Auch wird durch diese Vorgehensweise sichergestellt, daß die Reinigungsflüssigkeiten jeweils frisch zugeführt werden, daher keinerlei Kreislauf stattfindet, bei dem gegebenenfalls bereits verwendete Flüssigkeiten erneut genutzt werden.

Die einzelnen Schritte des erfindungsgemäßen Verfahrens können sowohl bei einer Innenreinigung als auch bei einer Außenreinigung des Handstücks vorgesehen sein, besonders bevorzugt wird das Handstück sowohl einer Innenreinigung als auch einer Außenreinigung unterzogen, wobei diese entweder gleichzeitig oder konsekutiv stattfinden können.

Ferner ist es möglich, daß ein Verfahren und eine entsprechende Vorrichtung vorgesehen werden, bei der jeweils ein einzelnes Handstück gereinigt wird, es ist jedoch auch möglich, ein Verfahren und eine zugehörige Vorrichtung vorzusehen, bei der mehrere Handstücke gleichzeitig gereinigt werden können.

Bei einem bevorzugten Verfahren wird als Vorspülflüssigkeit in Schritt A lediglich Wasser verwendet, wobei dieses Wasser dem Handstück beispielsweise aus einer Leitung direkt zugeführt werden kann, also im wesentlichen eine Leitungswasser-Temperatur aufweist, wobei das Leitungswasser nicht zusätzlich aufgeheizt wird. Typische Temperaturen für das Wasser in einem solches Verfahren liegen bei ca. 15°C bis 20°C. Dies hat zum einen den Vorteil, daß die Temperatur der Vorspülflüssigkeit deutlich unter der Koagulationstemperatur der Eiweiße liegt, zum anderen wird keine zusätzliche Heizenergie benötigt, so daß das Verfahren kostengünstig durchzuführen ist.

Es soll an dieser Stelle darauf hingewiesen werden, daß Wasser, beispielsweise Leitungswasser, durchaus mit automatischen Zusätzen, wie zum Beispiel Chlor oder Fluor, versetzt sein kann, wenn jedoch in dieser Beschreibung erwähnt wird, daß Wasser "ohne Zusätze" verwendet wird, ist darunter zu verstehen, daß keinerlei Zusätze im Rahmen des Reinigungsverfahrens oder in der Reinigungsvorrichtung, wie zum Beispiel Reinigungsmittel oder Oxidationsmittel, zugeführt werden.

Bei einer anderen Ausführungsform ist es jedoch auch möglich, daß die Vorspülflüssigkeit sowohl Wasser als auch ein Reinigungsmittel enthält, bevorzugt auch in diesem Schritt bereits ein enzymatisches Reinigungsmittel, wobei jedoch zumindest im Vorspül-Schritt A auch andere Reinigungsmittel eingesetzt werden können. Als Reinigungsmittel im Vorspülprozeß eignen sich besonders Detergentien, die diesen Vorspülprozeß erleichtern. Sie setzen insbesondere die Grenzflächenspannung der zu reinigenden Oberfläche, der Verunreinigungen und des Lösemittels, hier Wasser, herab. Insbesondere empfiehlt es sich, natürlich vorkommende oder synthetisch hergestellte Tenside oder Emulgatoren und Netzmittel zu verwenden.

Das Flüssigkeitsgemisch, das in Schritt B beim Spülen des Handstücks verwendet wird, umfaßt Wasser und enthält bevorzugt zumindest eine Enzymgruppe, wobei bevorzugt Proteasen eingesetzt werden. Besonders bevorzugt werden mehrere Enzymgruppen, besonders Proteasen und Lipasen verwendet, wobei bei einem anderen bevorzugten Verfahren auch Amylasen, zusätzlich oder an Stelle der vorgenannten Enzymgruppen, eingesetzt werden. Diese Enzymgruppen haben sich als besonders wirkungsvoll erwiesen und stellen zum einen ein hervorragendes Reinigungsergebnis sicher, zum anderen führen sie zu einer guten Konditionierung für den nachfolgenden Desinfektionsschritt.

Die Konzentration des Reinigungsmittels liegt bei einem bevorzugten Verfahren zwischen 0,1 % und 2 %, insbesondere 0,1 % bis 1 %, insbesondere zwischen 0,3 und 0,7 %, wobei festgestellt worden ist, daß eine optimale Konzentration bei ungefähr 0,5 % liegt.

Bevorzugt umfaßt das Flüssigkeitsgemisch zum Spülen des Handstücks neben dem Reinigungsmittel zusätzlich einen Entschäumer oder eine schaumhemmende Verbindung. Dies verhindert eine die Reinigungswirkung gegebenenfalls mindernde Schaumbildung, die insbesondere bei der Zersetzung der Proteine, insbesondere durch Proteasen, entstehen kann.

Bevorzugt umfaßt das in Schritt C zum Desinfizieren der Handstücke verwendete Flüssigkeitsgemisch Wasser und ein Oxidationsmittel mit mindestens einer Peroxidverbindung. Bevorzugt werden organische Peroxidverbindungen, zum Beispiel Perfruchtsäure oder Peressigsäure, verwendet, es ist aber auch möglich, zusätzlich oder an Stelle der organischen Peroxidverbindungen anorganische Peroxidverbindungen, zum Beispiel Wasserstoffperoxid, einzusetzen. Als ein besonders bevorzugtes Oxidationsmittel wird insbesondere Perbenzoesäure eingesetzt, die nicht so aggressiv ist wie manch anderes Oxidationsmittel, dennoch die gewünschten Ergebnisse und insbesondere die beschriebenen kombinatorischen Effekte erzielt. Es hat sich ferner herausgestellt, daß insbesondere organische Peroxide besonders stark auf die durch das enzymatische Reinigungsmittel hervorgerufene Konditionierung "reagieren" und daher besonders gute kombinatorische Reinigungs- und Desinfektionswirkungen zeigen.

Der Schritt B des Spülens des Handstückes mit einem Flüssigkeitsgemisch, das bevorzugt auch ein enzymatisches Reinigungsmittel umfaßt, findet bevorzugt in einem Temperaturbereich von 50°C bis 65°C statt, wobei Versuche ergeben haben, daß optimale Ergebnisse in einem Temperaturbereich von 55°C ± 2°C erzielt werden.

Auch für das Desinfizieren mit einem Flüssigkeitsgemisch, das Wasser und bevorzugt ein Oxidationsmittel umfaßt, wird ein bevorzugter Temperaturbereich von 50°C bis 65°C gewählt, wobei Versuche gezeigt haben, daß ein Temperaturbereich von 62°C ± 2°C zu optimalen Ergebnissen im Hinblick auf die Konditionierung durch das Spülen und die Nutzung der Konditionierung durch das Desinfizieren geführt hat.

Für das Vorspülen des Handstücks mit einer Vorspülflüssigkeit, insbesondere mit Wasser, wie oben erwähnt bevorzugt mit Leitungswasser und bei entsprechender Leitungswasser-Temperatur, wird bevorzugt auch der zur Verfügung gestellte Druck des Leitungswassers verwendet, insbesondere in einem Bereich von 2 bis 6 bar, insbesondere 3 bis 4 bar.

Für den Schritt B und das Spülen mit einem Flüssigkeitsgemisch, das Wasser und ein, bevorzugt enzymatisches, Reinigungsmittel umfaßt, werden bevorzugt Drücke zwischen 1 bar und 1,5 bar verwendet, wobei festgestellt worden ist, daß optimale Ergebnisse bei etwa 1,1 bar erzielt werden. Ein Druck von etwa 1,1 bar hat sich insbesondere deshalb als bevorzugt herausgestellt, da er einen optimalen Wert im Hinblick auf den Reinigungseffekt einerseits und den Wasserverbrauch andererseits darstellt, d.h. der Druck ist hoch genug, daß ein sehr guter Reinigungseffekt erzielt wird, gleichzeitig jedoch relativ gering, um den Wasserverbrauch ebenfalls gering zu halten. Gleiche Drücke werden bevorzugt für das Desinfizieren des Handstücks mit Wasser und einem Oxidationsmittel verwendet, auch hier haben Versuche gezeigt, daß ein Druck von etwa 1,1 bar zu optimalen Ergebnissen führt.

Bevorzugt wird das erfindungsgemäße Verfahren durch zusätzliche Verfahrensschritte ergänzt, die vor oder nach den oben genannten Schritten A, B und C durchgeführt werden könne, wobei es ferner möglich ist, Zwischenschritte einzufügen.

Bei einer besonders bevorzugten Ausführungsform wird zwischen dem Schritt A und dem Schritt B ein zusätzlicher Schritt A1 eingefügt, bei dem das Handstück mit einem Flüssigkeitsgemisch gespült wird, das Wasser und ein Reinigungsmittel enthält, wobei bevorzugte Temperaturen von 30°C bis 40°C, insbesondere ungefähr 35°C, verwendet werden, wobei bevorzugt in Schritt A das Vorspülen des Handstücks bei Leitungswasser-Temperatur, ungefähr 15°C bis 20°C, durchgeführt wird.

Dieses schrittweise Erhöhen der Temperatur führt zu besonders guten Reinigungsleistungen und verlängert die gesamte Behandlungsdauer des oder der Handstücke nur unwesentlich.

Bei einem weiteren besonderen Verfahren wird zwischen Schritt B und C ein weiterer Verfahrensschritt B1 eingeführt, der ein Zwischenspülen mit Wasser umfaßt, wobei dem Wasser keine Zusätze, wie beispielsweise ein enzymatisches Reinigungsmittel oder ein Oxidationsmittel oder ein sonstiges Reinigungsmittel, zugesetzt sind. Bevorzugt wird dieses Zwischenspülen bei einer Temperatur von 60°C bis 70°C durchgeführt.

Bei einem weiteren bevorzugten Verfahren findet nach Schritt C ein Nachspülschritt statt, der als Schritt C1 bezeichnet wird, wobei auch hier Wasser, bevorzugt ohne irgendwelche Zusätze, verwendet wird. Die Temperaturen des Nachspülschritts liegen bei maximal 70°C und bevorzugt unter 60°C und können während dieses Schritts abnehmen, um die Temperatur auch des Handstücks selbst langsam zu senken.

Bevorzugt umfaßt das Verfahren ferner einen Trocknungsschritt mit warmer Luft, Schritt D, wobei bevorzugte Temperaturen zwischen 80°C und 95°C und insbesondere im Bereich von ca. 90°C liegen. Bei einem anderen bevorzugten Verfahren wird in Schritt D lediglich Luft zugeführt, ohne diese speziell aufzuheizen, wobei für den Trockenschritt dann die Restwärme des Handstücks aufgrund der vorhergehenden Reinigungsschritte verwendet werden kann. Dies hat auch den Vorteil, dass bereits beim Trocknungsschritt das Handstück abgekühlt wird und so schnell wiederverwendet werden kann.

Ferner kann das Verfahren einen zusätzlichen Schritt E umfassen, bei dem Teile des Handstücks mit einem Schmiermittel versorgt werden. Dadurch werden insbesondere bewegliche Teile der Handstücke besonders gepflegt, was die Lebensdauer der Handstücke erhöht und ein einwandfreies Arbeiten ermöglicht.

Gemäß einer Ausgestaltung des erfindungsgemäßen Verfahrens wird bei zumindest einem der oben genannten Schritte zumindest teilweise Druckluft zugeführt. Bevorzugt wird dem Flüssigkeitsstrom in gepulster Form Druckluft zugeführt, was insbesondere den Vorteil hat, daß die mechanische Wirkung der Reinigung während der einzelnen Schritte verbessert wird. Die Druckimpulse können beispielsweise mit einer Frequenz von 0,5 Hz bis 2 Hz und mit einem Druck im Bereich von 1 bar bis 1,1 bar durchgeführt werden. Die Druckimpulse in der bevorzugten Frequenz und mit den oben genannten Drücken stellen sicher, daß eine pulsierende Bewegung erreicht wird, wobei bereits die genannten relativ geringen Drücke ausreichen, höhere Drücke verbessern wider Erwarten nicht merklich die Reinigungsergebnisse.

Die Erfindung betrifft ferner eine Vorrichtung zum Durchführen eines Verfahrens, wie es oben beschrieben worden ist, wobei die Vorrichtung eine Aufnahmevorrichtung zur Aufnahme mindestens eines medizinischen Handstücks, einen Vorratsbehälter für ein Reinigungsmittel, bevorzugt einen Vorratsbehälter für ein Oxidationsmittel, einen Vorratsbehälter für Wasser oder einen Anschluß für eine Wasserversorgung, eine Steuer-/Dosiervorrichtung und eine Vorrichtung zum Zuführen von Wasser, Reinigungsmittel und ggf. Oxidationsmittel in die Steuer-/Dosiereinrichtung sowie eine Vorrichtung zum Zuführen der verwendeten Medien, einzeln oder in gemischter Form, und eine Heizvorrichtung umfaßt.

Als Heizvorrichtung wird bevorzugt ein Durchlauferhitzer verwendet, was ebenfalls das oben beschriebene Durchlaufprinzip bestmöglichst unterstützt.

Bevorzugt umfaßt die Vorrichtung ferner ein Druckluftreservoir oder einen Anschluß an eine Druckluftversorgung.

Die Vorrichtung umfaßt bevorzugt mindestens ein Anschlußelement zum Zuführen der Flüssigkeiten und/oder der Luft in das Innere des mindestens einen Handstücks, wobei die Vorrichtung ferner bevorzugt eine Abgabevorrichtung zur Abgabe von Flüssigkeit und/oder Luft auf Außenbereiche des Handstücks zur Außenreinigung, beispielsweise Düsen, umfaßt. Ferner umfaßt die Vorrichtung eine Abflußvorrichtung, die auch zum kontinuierlichen Abführen der verwendeten Flüssigkeit geeignet ist.

Eine besonders bevorzugte Ausführungsform der Vorrichtung umfaßt ferner eine Vorrichtung zur Abgabe von Ultraschall, insbesondere zum Unterstützen des Desinfizierens oder des Sterilisierens der Außenbereiche des Handstücks oder der Handstücke. Ferner kann bevorzugt ein Zerstäuber für eine Zerstäubung des Schmiermittels vorgesehen sein, wobei der Zerstäuber bevorzugt mit Druckluft arbeitet.

Die Vorrichtung kann so ausgebildet sein, daß lediglich ein Handstück eingesetzt und gereinigt werden kann, bevorzugt sind jedoch Vorrichtungen, bei denen gleichzeitig mehrere Handstücke, beispielsweise drei Handstücke, eingesetzt werden können und gereinigt werden können.

Das erfindungsgemäße Verfahren und die erfindungsgemäße Vorrichtung können bei einem gleichzeitigen Einsatz von mehreren Handstücken so ausgelegt sein, daß sämtliche Handstücke gleichzeitig gereinigt werden, d.h. sämtliche Verfahrensschritte simultan für alle eingesetzten Handstücke durchgeführt werden, besonders bevorzugt ist es jedoch, daß die einzelnen Verfahrensschritte konsekutiv ausgeführt werden, jedoch pro Verfahrensschritt erst für alle eingesetzten Handstücke, so daß zumindest für den Benutzer eine quasi-gleichzeitige Reinigung und Desinfizierung bzw. Behandlung der Handstücke durchgeführt wird.

Diese und weitere Merkmale und Vorteile der Erfindung werden anhand der beigefügten Zeichnungen noch deutlicher werden:
- Fig. 1: zeigt schematisch eine Ausführungsform einer erfindungsgemäßen Vorrichtung zur Durchführung eines erfindungsgemäßen Verfahrens;
- Fig. 2: zeigt beispielhaft eine mögliche Ausführungsform des erfindungsgemäßen Verfahrens mit den einzelnen Verfahrensschritten in zeitlicher Abfolge.

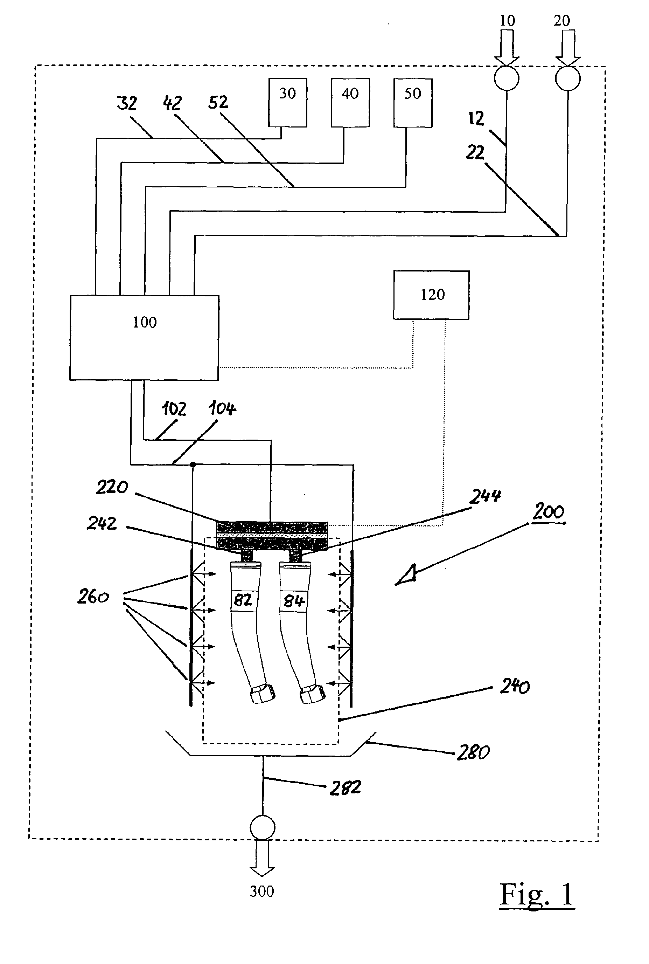

Fig. 1 zeigt schematisch eine Ausführungsform einer erfindungsgemäßen Vorrichtung zur Durchführung eines erfindungsgemäßen Verfahrens. Die Vorrichtung umfaßt einen Vorratsbehälter 30 für ein Schmiermittel, insbesondere Öl, einen Vorratsbehälter 40 für ein, bevorzugt enzymatisches, Reinigungsmittel und einen Vorratsbehälter 50 für ein Oxidationsmittel. Ferner umfaßt die Vorrichtung einen Anschluß 10 für eine Wasserversorgung, hier eine Leitungswasserversorgung, und einen Anschluß 20 für eine Druckluftversorgung.

Die Anschlüsse 10 und 20 sowie die Vorratsbehälter 30, 40 und 50 sind über Leitungen 12, 22, 32, 42 und 52 mit einer Steuer-/Dosiereinrichtung 100 verbunden. Die Steuer-/Dosiereinrichtung 100 wird von einer Computervorrichtung 120 gesteuert.

Die Steuer-/Dosiereinrichtung 100 umfaßt ein Ventil, das den Durchfluß des Wassers ermöglichen oder unterbrechen kann bzw. welches die Durchflußmenge des Wassers durch die Leitung 12 steuern bzw. regeln kann. Die Steuer-/Dosiereinrichtung 100 umfaßt ferner ein zweites Ventil, das den Durchfluß der Luft in der Leitung 22 ermöglichen, unterbrechen bzw. die Durchflußmenge steuern bzw. regeln kann.

Die Steuer-/Dosiereinrichtung umfaßt ferner insgesamt drei Pumpenvorrichtungen, wobei jeweils eine Pumpenvorrichtung zur Förderung der Flüssigkeiten aus den Vorratsbehältern 30, 40 und 50 vorgesehen ist. Die Pumpen werden von der Steuer-/Dosiereinrichtung 100 und dem Computer 120 gesteuert, so daß ihre Leistung und damit die Fördermenge einstellbar ist.

In der Steuer-/Dosiereinrichtung 100 münden die Leitungen 42 und 52 für das Reinigungsmittel und das Desinfektionsmittel in die Leitung 12 für die Wasserzuführung, wobei bevorzugt im Mündungsbereich Mischkammern vorgesehen sind. Dadurch wird insbesondere das kontinuierliche Durchlaufverfahren ermöglicht, wobei bevorzugt kontinuierlich Wasser durch die Leitung 12, die Steuer-/Dosiereinrichtung 100 und die Leitung 102 bzw. 104 auf die Handstücke bzw. in die Handstücke geleitet werden kann, wobei wenn erforderlich das Reinigungsmittel und/oder das Desinfektionsmittel zugeführt werden.

Eine Leitung 102 verbindet die Steuer-/Dosiereinrichtung 100 mit einem Medienübergabeblock 220, wobei die Medien, die durch die Leitungen 12, 22, 32, 42 und 52 der Steuer-/Dosiereinrichtung zugeführt werden, zusammengeführt und an den Medienübergabeblock 220 weitergeleitet werden können.

Die zu reinigenden Handstücke 82 und 84 können über Anschlußvorrichtungen bzw. Nasen 242 und 244 an den Medienübergabeblock 220 angeschlossen werden. Dies kann direkt geschehen, es ist jedoch auch möglich, wie in der hier gezeigten Ausführungsform, eine Kassette 240 vorzusehen, die die Nasen 242 und 244 aufweist, so daß die Handstücke 82 und 84 an die Nasen 242 und 244 der Kassette 240 angeschlossen werden können, woraufhin dann die gesamte Kassette 240 an den Medienübergabeblock 220 angeschlossen wird.

Im Medienübergabeblock 220 sind bei dieser besonders bevorzugten Ausführungsform noch einmal Mischkammern vorgesehen, es ist jedoch möglich, auf diese Mischkammern zu verzichten, nachdem bereits Mischkammern in der Steuer-/Dosiereinrichtung vorgesehen sind.

Der Vollständigkeit halber soll an dieser Stelle darauf hingewiesen werden, daß es durchaus auch möglich ist, daß einige oder alle Leitungen 12, 22, 32, 42 und 52 bis in den Medienübergabeblock 220 weitergeleitet werden, wobei die Zuführung der Medien, wie oben beschrieben, durch die Steuer-/Dosiereinrichtung 100 geregelt wird, in welchem Falle Mischkammern ausschließlich in dem Medienübergabeblock 220 vorgesehen sind. Bei einer besonders bevorzugten Ausführungsform sind die Steuer-/Dosiereinrichtungen 100 und der Medienübergabeblock 220 als integrale Vorrichtung oder einteilig ausgebildet. Dies führt zu einem besonders kompakten Gesamtsystem.

Eine zusätzliche Leitung 104 befördert die Medien, die durch die Leitungen 12, 22, 42 und 52 an die Steuer-/Dosiereinrichtung 100 geleitet werden, zu mehreren Düsenvorrichtungen 260 weiter, die zur Außenbehandlung der Handstücke 82, 84 die Medien auf die Außenseite der Handstücke sprühen. Eine Außenbehandlung mit einem Schmiermittel ist nicht erforderlich, daher mündet die Leitung 32 nicht in die Leitung 104. Die Düsen 260 können als Teil des Reinigungsgeräts vorgesehen sein und, im Falle des Vorsehens einer Kassette 240, durch Öffnung in der Kassette in das Innere der Kassette reichen. Es ist jedoch auch möglich, daß die Düsen als Teil der Kassette ausgeführt sind.

Zum Schmieren wird Schmiermittel aus dem Behälter 30 durch die Leitungen 32 und 102 in die Nasen 242 und 244 gefördert. Bei einer bevorzugten Ausführungsform ist es möglich, daß insbesondere die Leitung für das Schmiermittel bis in den Medienübergabeblock getrennt von der oder den Leitungen für die anderen Medien geführt wird. Bevorzugt mündet jedoch in der Steuer-/Dosiereinrichtung 100 zumindest eine Abzweigung der Druckluftleitung 22 in die Schmiermittelleitung, so daß das Schmiermittel zerstäubt werden kann.

Die aus den Handstücken 82 und 84 austretenden Medien sowie die Medien, die zur Außenreinigung verwendet werden, fließen in eine Auffangvorrichtung 280, die bevorzugt mit einem Trichter versehen ist, von der aus die Medien über eine Abflußleitung 282 und einen Abfluß 300 bevorzugt über eine Pumpe abgeführt werden. Das hier dargestellte Reinigungsgerät besitzt mit Ausnahme der Vorratsbehälter 30, 40 und 50 keine flüssigkeits- oder druckdichte Behälter, in denen die zur Reinigung verwendeten Flüssigkeiten stehen bzw. gestaut werden müssen und wo sich Partikel, Schmiermittel und Mikroorganismen ablagern können. Es kommt auch zu keiner Mehrfachverwendung bzw. Rückführung von Flüssigkeiten, wodurch vorteilhafterweise die Gefahr der Kontamination bzw. Re-Kontamination von Handstücken durch das Reinigungsgerät selbst vermieden wird.

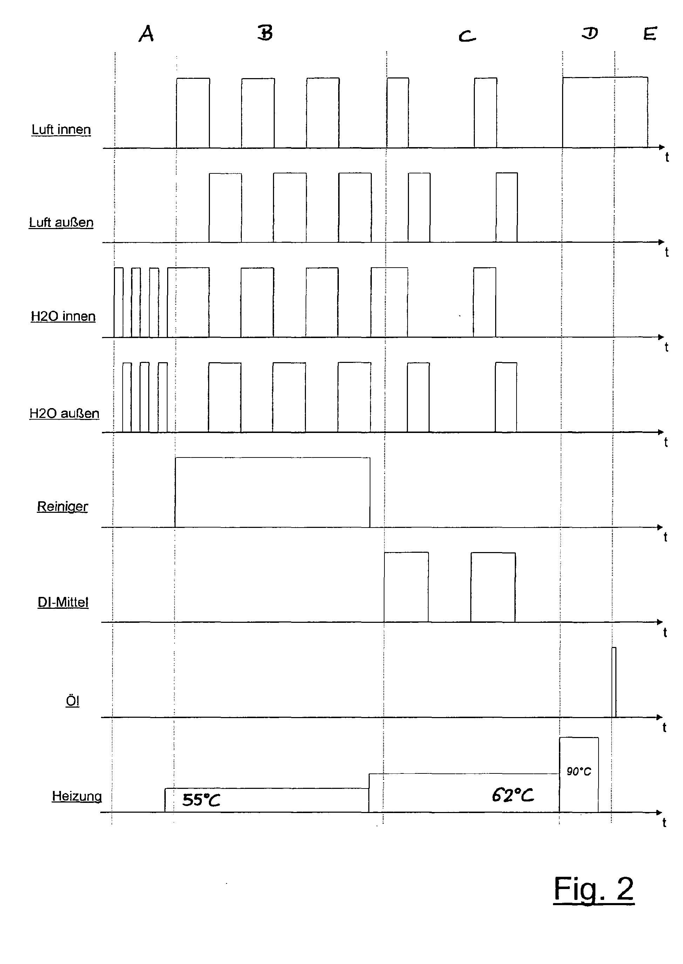

Fig. 2 zeigt schematisch einen bevorzugten Gesamtprozeß, in dem die Zuführung der einzelnen Medien in zeitlicher Abfolge dargestellt ist.

Die hier gezeigte besondere Ausführungsform des erfindungsgemäßen Verfahrens umfaßt insgesamt fünf Schritte, eine Vorreinigung bzw. einen Vorspül-Schritt A, einen ReinigungsSchritt B, einen Desinfektions-Schritt C, einen Trocknungs-Schritt D und einen Schmier-/Ausblas-Schritt E.

Im Vorspül-Schritt A wird lediglich Wasser zugeführt, wobei Wasser sowohl durch das Innere der Handstücke geleitet wird als auch auf die Außenseite der Handstücke gesprüht wird, wobei dies abwechselnd geschieht.

Im nachfolgenden Reinigungsschritt B wird sowohl Druckluft als auch Wasser zugeführt, abwechselnd wieder in das Handstückinnere bzw. auf den Außenbereich des oder der Handstücke, wobei kontinuierlich das enzymatische Reinigungsmittel zugeführt wird. Bei diesem Schritt werden die zugeführten Medien auch zusätzlich aufgewärmt, bei der hier gezeigten Ausführungsform auf ca. 55°C.

Der nachfolgende Desinfektions-Schritt C läuft verfahrensmäßig ähnlich wie der vorher beschriebene Schritt B, wobei an Stelle des enzymatischen Reinigers ein Oxidationsmittel zugeführt wird. Der Desinfektions-Schritt C wird zweimal mit einer kurzen Unterbrechung durchgeführt, wobei bei dem hier gezeigten Verfahren eine bevorzugte Temperatur von ca. 62°C eingestellt wird.

Nachfolgend wird ein Trocknungs-Schritt D durchgeführt, bei dem Druckluft auf ca. 90°C erhitzt und durch den Innenbereich des Handstücks bzw. der Handstücke geleitet wird.

In einem abschließenden Schmier- und Ausblas-Schritt E wird über Druckluft ein Schmiermittel, insbesondere Öl, dem Handstückinneren zugeführt, wobei Öl nur für einen kurzen Zeitraum zugeführt wird, während das Ausblasen über einen längeren Zeitraum stattfindet, wie deutlich in Fig. 2 ersichtlich ist.

Die einzelnen dargestellten Verfahrensschritte können in ihrer Länge variiert werden. In Schritt A dauern die einzelnen Blöcke des Zuführens von Wasser sowohl im Innenbereich als auch im Außenbereich bevorzugt 3 bis 5 Sekunden, die einzelnen Blöcke des Zuführens von Luft und Wasser in Schritt B dauern bevorzugt etwa 5 bis 20 Sekunden.

Die einzelnen Blöcke im Desinfektions-Schritt C dauern bei einer bevorzugten Ausführungsform ebenfalls etwa 20 bis 40 Sekunden, während der Trocknungs-Schritt D bevorzugt etwa 10 bis 20 Sekunden dauert. Es soll an dieser Stelle darauf hingewiesen werden, daß der Schritt C auch über einen längeren Zeitraum durchgeführt werden kann, was dann zu besseren Ergebnissen bis hin zu einer Sterilisation führt. Bei Schritt D ist es ferner möglich, diesen zweiteilig zu gestalten, wobei in einem ersten Schritt die Lufterhitzung eingeschaltet ist, wie auch in Fig. 2 ersichtlich, wobei dieser erste Schritt etwa 10 bis 15 Sekunden dauert, während in einem zweiten Schritt die Lufterhitzung ausgeschaltet wird und bevorzugt das Ausblasen weitere 5 bis 30 Sekunden andauert.

Im Schritt E wird nur über einen ganz kurzen Zeitraum das Schmiermittel zudosiert, während der Schritt des Ausblasens bevorzugt 10 bis 20 Sekunden andauert.

Mit einem erfindungsgemäßen Verfahren, wie es in einer besonderen Ausführungsform in Fig. 2 dargestellt ist, kann daher das gesamte Verfahren mit hervorragenden Ergebnissen aber in sehr kurzer Zeit abgeschlossen werden, der Gesamtzyklus liegt bei beispielsweise drei Handstücken in einem Zeitrahmen von 8 bis 12 Minuten.

Die in der vorstehenden Beschreibung, den Ansprüchen und den Zeichnungen offenbarten Merkmale können sowohl einzeln als auch in beliebiger Kombination für die Ausführung der Erfindung in ihren verschiedenen Ausgestaltungen von Bedeutung sein.

## Patentansprüche

1. Verfahren zur Aufbereitung eines medizinischen Instruments (82, 84), das die folgenden Schritte umfaßt:
A) Vorspülen des Instruments (82, 84) mit einer Vorspülflüssigkeit,
B) Spülen des Instruments (82, 84) mit einem Flüssigkeitsgemisch, das Wasser und ein Reinigungsmittel umfaßt
C) Desinfizieren des Instruments (82, 84) mit einem Flüssigkeitsgemisch, das Wasser umfasst,
wobei die einzelnen Verfahrensschritte kontinuierlich durchgeführt werden, wobei Wasser als Trägermedium kontinuierlich zugeführt wird, zum Vorspülen, Spülen und Desinfizieren des zu reinigenden Instruments (82, 84) verwendet und kontinuierlich abgeführt wird.

2. Verfahren nach Anspruch 1, wobei dem Trägermittel Wasser während des Spülens, Schritt B, das enzymatische Reinigungsmittel und während des Desinfizierens, Schritt C, das Oxidationsmittel zugeführt wird, bevor das Trägermittel auf das zu reinigende Instrument (82, 84) wirkt.

3. Verfahren nach Anspruch 1, **dadurch gekennzeichnet, daß** es sich bei der in Schritt A verwendeten Vorspülflüssigkeit um Wasser handelt oder die in Schritt A verwendete Vorspülflüssigkeit Wasser und ein Reinigungsmittel enthält.

4. Verfahren nach einem der vorhergehenden Ansprüche, **dadurch gekennzeichnet, daß** das in Schritt B verwendete Flüssigkeitsgemisch zum Spülen des Instruments (82, 84) mindestens eine Enzymgruppe enthält.

5. Verfahren nach einem der vorhergehenden Ansprüche, **dadurch gekennzeichnet, daß** das in Schritt B verwendete Flüssigkeitsgemisch zum Spülen des Instruments (82, 84) zusätzlich einen Entschäumer oder eine schaumhemmende Verbindung umfaßt.

6. Verfahren nach einem der vorhergehenden Ansprüche, **dadurch gekennzeichnet, daß** Schritt A mit einer Vorspülflüssigkeit mit einer Temperatur von 15°C bis 20°C durchgeführt wird, wobei ferner zwischen Schritt A und Schritt B ein zusätzlicher Verfahrensschritt A1 durchgeführt wird, bei dem das Instrument (82, 84) mit einem Flüssigkeitsgemisch gespült wird, das Wasser und ein Reinigungsmittel enthält und dessen Temperatur zwischen 30°C und 40°C liegt.

7. Verfahren nach einem der vorhergehenden Ansprüche, **dadurch gekennzeichnet, daß** zwischen Schritt B und Schritt C ein weiterer Verfahrensschritt B1 durchgeführt wird, bei dem es sich um eine Zwischenspülung handelt und bei dem das Instrument (82, 84) mit Wasser in einem Temperaturbereich von 60°C bis 70°C gespült wird.

8. Verfahren nach einem der vorhergehenden Ansprüche, **dadurch gekennzeichnet, daß** nach Schritt C ein Nachspülschritt C1 durchgeführt wird, bei dem das Instrument mit Wasser mit einer maximalen Temperatur von 70°C gespült wird.

9. Verfahren nach einem der vorhergehenden Ansprüche, **dadurch gekennzeichnet, daß** das Verfahren einen zusätzlichen Trocknungsschritt D umfaßt, bei dem das Instrument (82, 84) mit warmer Luft behandelt wird.

10. Verfahren nach einem der vorhergehenden Ansprüche, **dadurch gekennzeichnet, daß** das Verfahren zusätzlich einen Verfahrensschritt E umfaßt, bei dem dem Instrument (82, 84) Schmiermittel zugeführt wird.

11. Verfahren nach einem der vorhergehenden Ansprüche, **dadurch gekennzeichnet, daß** in einem der oben genannten Schritte zumindest teilweise Druckluft zugeführt wird.

12. Vorrichtung zum Durchführen eines Verfahrens nach einem der vorhergehenden Ansprüche, das umfaßt:
- eine Aufnahmevorrichtung (242, 244) zur Aufnahme mindestens eines medizinischen Instruments (82, 84)
- einen Vorratsbehälter (40) für ein Reinigungsmittel,
- einen Vorratsbehälter für Wasser oder eine Anschlußvorrichtung (10) für einen Anschluß an eine Wasserversorgung,
- eine Steuer-/Dosiereinrichtung (100),
- eine Vorrichtung zum Zuführen von Wasser, Reinigungsmittel und/oder Oxidationsmittel in die Steuer-/Dosiereinrichtung (100),
- eine Vorrichtung zum kontinuierlichen Zuführen von Medien an und/oder in das mindestens eine medizinische Instrument (82, 84),
- eine Heizvorrichtung, und
- eine Vorrichtung zum kontinuierlichen Abführen von Medien.

13. Vorrichtung nach Anspruch 12, die ein Anschlußelement (242, 244) zum Zuführen der Medien in das Innere des Instruments (82, 84) umfaßt.

14. Vorrichtung nach Anspruch 12 oder 13, die ferner eine Abgabevorrichtung (260) zur Abgabe der Medien auf einen Außenbereich des Instruments (82, 84) umfaßt.

15. Vorrichtung nach einem der Ansprüche 12 bis 14, die ferner eine Abflußvorrichtung (280, 282, 300) zum kontinuierlichen Abfluß der Medien umfaßt.
